# EUROPÄISCHE PATENTSCHRIFT

(11) **EP 3 844 537 B1**
(45) Veröffentlichungstag und Bekanntmachung des Hinweises auf die Patenterteilung: **18.06.2025**
(21) Anmeldenummer: 19753334.2
(22) Anmeldetag: 12.08.2019
(51) Int. Cl.: G02B 5/32, G02B 27/00, G02B 27/01, G06F 3/01, A61B 3/113, A61B 3/107, G01B 9/02, G01S 7/4912

(54) **VERFAHREN ZUM ERMITTELN EINER BLICKRICHTUNG EINES AUGES**
METHOD FOR ASCERTAINING A VIEWING DIRECTION OF AN EYE
PROCÉDÉ POUR DÉTERMINER UNE DIRECTION DU REGARD D'UN OEIL

(30) Priorität: 29.08.2018 DE 102018214637
(43) Veröffentlichungstag der Anmeldung: 07.07.2021
(73) Patentinhaber: Robert Bosch GmbH, 70442 Stuttgart (DE)
(72) Erfinder: FIESS, Reinhold, 77770 Durbach (DE); PETERSEN, Andreas, 71672 Marbach (DE); SCHLEBUSCH, Thomas Alexander, 71272 Renningen (DE)
(86) Internationale Anmeldenummer: PCT/EP2019/071562
(87) Internationale Veröffentlichungsnummer: WO 2020/043472

(56) Entgegenhaltungen:
- WO-A1-2019/126150
- DE-A1- 102016 226 294
- US-A1- 2016 349 515
- US-A1- 2017 109 562
- US-A1- 2017 115 483
- US-B2- 7 657 062
- US-B2- 9 529 191
- THOMAS TAIMRE ET AL: "Laser feedback interferometry: a tutorial on the self-mixing effect for coherent sensing", ADVANCES IN OPTICS AND PHOTONICS, vol. 7, no. 3, 20 August 2015 (2015-08-20), pages 570, XP055595627, DOI: 10.1364/AOP.7.000570

## Beschreibung

Die vorliegende Erfindung betrifft ein Verfahren zum Ermitteln einer Blickrichtung eines Auges, eine Projektionsvorrichtung für eine Datenbrille, eine Datenbrille, ein Computerprogramm, ein maschinenlesbares Speichermedium sowie ein elektronisches Steuergerät.

### Stand der Technik

Aufgabe der Okulografie, Blickerfassung oder auch engl. eye-tracking ist es, die Bewegung der Augen zu erfassen und die Blickrichtung zu ermitteln. Die Technologie findet breite Anwendung in der Konsumentenforschung, z.B. bei der Beobachtung des Leseflusses zur Optimierung der Platzierung von Werbung.

Bei Datenbrillen, wie z.B. bei Helmet-Mounted- bzw. Head-Mounted- (HMD) oder Head-Worn-Displays (HWD), kommt die Technik auch zum Einsatz, um abhängig von der Blickrichtung des Benutzers den dargestellten Bildinhalt anzupassen. Einerseits kann beispielsweise zur elektrischen Leistungsreduktion die Darstellung hochaufgelöster Bildinhalte auf den Bereich des schärfsten Sehens begrenzt und peripher geringer aufgelöste Inhalte dargestellt werden. Andererseits können vor allem bei Augmented Reality Systemen, welche teil- oder volltransparente Systeme sind, die eine natürliche Wahrnehmung des Umfelds mit der Darstellung virtueller Inhalte kombinieren, Informationen, Ergänzungen und Hinweise aus Gründen der Übersichtlichkeit nur für betrachtete Objekte eingeblendet werden.

Technologisch können die verfügbaren Systeme in folgende Kategorien eingeteilt werden:
Kamerabasierte Systeme: Eine Kamera ist vom Brillengestell auf das Auge gerichtet. Bei einigen Systemen kommen zusätzlich ein oder mehrere Infrarot-Lichtquellen hinzu, die gegebenenfalls moduliert betrieben werden. Durch ein Augenmodell, kontrastbasierte Detektion der Pupille (dark vs. bright pupil), Limbus-Tracking (Kontrastgrenze zwischen Cornea und Sclera) oder Lokalisierung des Hornhautreflexes wird die Blickrichtung aus den Bilddaten ermittelt. Es sind auch Systeme beschrieben worden, die über strukturiertes Licht ein Muster (meist Punktwolken- oder Linienmuster) auf das Auge projizieren und über die charakteristische Formänderung im Bereich der Cornea gegenüber dem Bereich der Sclera die Blickrichtung ermitteln.

Gescannte Lasersysteme: Eine im Brillengestell integrierte Laserquelle wird beispielsweise durch einen Mikro-Elektro- Mechanischen (MEMS) Mikrospiegel über das Auge geführt. Es sind Verfahren bekannt, die basierend auf dem Hornhautreflex die Blickrichtung ermitteln.

Elektrische Messung: Die durch lonenverschiebungen bei Muskelkontraktion entstehenden messbaren elektrischen Felder können als sogenanntes Elektromyogramm aufgezeichnet werden. Kommt das Verfahren im Bereich der Augen zum Einsatz, spricht man von Elektro-Oculogramm. Durch Signalverarbeitung der abgeleiteten elektrischen Signale kann ebenfalls auf die Augenbewegung geschlossen werden Elektro-Magnetische Messung: Befindet sich eine Spule im Magnetfeld einer anderen Spule, ist die magnetische Kopplung der beiden Spulen von der Ausrichtung zueinander abhängig. Wird eine Spule im Brillengestell und eine weitere Spule in einer Kontaktlinse integriert, ist ebenfalls eine Ermittlung der Blickrichtung möglich.

Die DE 10 2016 226 294 A1 beschreibt ein Verfahren zur Bestimmung der Brechkraft einer Linse in einem Auge. In diesem wird ein Laserstrahl über wenigstens eine Strahlablenkeinheit und über wenigstens ein Einkopplungselement auf die Linse des Auges umgeleitet. Auf der Basis einer Auswertung der zurückgestreuten und/oder reflektierten Strahlung, insbesondere auf der Basis einer optischen Feedback-Interferometrie, wird eine optische Weglänge bestimmt und daraus auf die Brechkraft der Linse geschlossen. Die Auswertung der zurückgestreuten und/oder reflektierten Strahlung erfolgt insbesondere auf der Basis einer optischen Feedback-Interferometrie. Das dem Verfahren zugrunde liegende Messprinzip basiert dabei vorzugsweise auf der auch als self-mixing-interference (SMI) bezeichneten Methode.

### Offenbarung der Erfindung

Das Verfahren dient dem Ermitteln einer Blickrichtung eines Auges. Hierbei handelt es sich insbesondere um ein Auge eines Nutzers einer Datenbrille.

Unter einer Datenbrille kann ein HMD verstanden werden. Unter dem Begriff Datenbrille soll ebenfalls eine Videobrille, ein Helmdisplay oder ein VR-Helm verstanden werden.

Die Blickrichtung des Nutzers ist hierbei gegeben durch einen Aufpunkt, welcher z.B. die Mitte der Pupille sein kann, und einen Richtungsvektor, entlang welchem das Auge sieht, welcher z.B. gegeben sein kann durch einen Vektor durch die Mitte der Pupille und einem Punkt auf der Retina, z.B. dem gelben Fleck. Der Aufpunkt kann sich z.B. durch Verschiebungen der Brille relativ zum Auge verändern.

Bei dem Verfahren wird ein von einer Laserquelle emittierter Laserstrahl über ein Reflexionselement und ein Umlenkelement über mindestens zwei Scanpunkte auf dem Auge, d.h. der Oberfläche des Auges, gescannt. Die mindestens zwei Scanpunkte liegen auf der Sklera und/oder auf der Iris und/oder auf der Pupille des Auges.

Das Reflexionselement dient dem Reflektieren des Lichtstrahls auf das Umlenkelement. Unter einem Reflexionselement kann beispielsweise ein Spiegel, insbesondere ein Mikrospiegel oder ein Array aus Mikrospiegeln, oder ein Hologramm verstanden werden. Mittels des Reflexionselements kann ein Strahlengang des Lichtstrahls an gegebene Raumverhältnisse angepasst werden. Beispielsweise kann das Reflexionselement als Mikrospiegel realisiert sein. Der Mikrospiegel kann beweglich ausgeformt sein, etwa eine um zumindest eine Achse neigbare Spiegelfläche aufweisen. Ein solches Reflexionselement bietet den Vorteil einer besonders kompakten Bauform. Es ist ferner vorteilhaft, wenn das Reflexionselement ausgebildet ist, um einen Einfallswinkel und, zusätzlich oder alternativ, einen Auftreffpunkt des Lichtstrahls auf dem Umlenkelement zu ändern. Dadurch kann das Umlenkelement flächig, insbesondere etwa in Zeilen und Spalten, mit dem Lichtstrahl überstrichen werden.

Das Umlenkelement kann ein holografisches Element oder ein Freiformspiegel sein.

Unter einem holografischen Element kann beispielsweise ein holografischoptisches Bauelement, kurz HOE, verstanden werden, das beispielsweise die Funktion einer Linse, eines Spiegels oder eines Prismas erfüllen kann. Je nach Ausführungsform kann das holografische Element für bestimmte Wellenlängen (Lichtfarben) und Einfallswinkel selektiv sein.

Verfügbare photoempfindliche Materialien (z.B. Photopolymerfilme) zur Erzeugung von holografischen Elementen sind nur im sichtbaren Wellenlängenbereich belichtbar. Von großem Vorteil wäre aber für das Verfahren der Einsatz von holografischen Elementen im Nahen Infrarotbereich, damit das Messverfahren für den Nutzer nicht sichtbar ist und um z.B. die Informationsdarstellung einer Datenbrille dadurch nicht zu stören. Durch geeignete Maßnahmen, wie z.B. Winkelvorhalt und/oder optische Immersionsaufbauten kann das photoempfindliche Material mit einer geeigneten Funktion im sichtbaren Wellenlängenbereich so belichtetet werden, dass das holografische Element bei Verwendung im Nahen Infrarot die gewünschte optische Zielfunktion besitzt.

Holografische Elemente können insbesondere durch optische Kopierprozesse in großer Stückzahl gefertigt werden. Erfüllt das holografische Element optische Funktionen, die mit einfachen Punkt- oder kollimierten Lichtquellen in das photoempfindliche Material belichtet werden können, gestaltet sich der Kopierprozess (Fertigungsprozess) wesentlich einfacher. Dadurch kann das holografische Element sehr kostengünstig hergestellt werden.

Das holografische Element kann transparent sein. Dadurch können Bildinformationen am Brillenglas mit der Umwelt überlagert werden.

Bei dem Verfahren wird ein von einer Laserquelle emittierter Laserstrahl über ein Reflexionselement und ein Umlenkelement über mindestens zwei Scanpunkte auf dem Auge gescannt.

Bevorzugt wird der Laserstrahl über eine Vielzahl von Scanpunkten gescannt.

Hierbei sind die Scanpunkte auf der Oberfläche des Auges so verteilt, dass die die Blickrichtung des Auges ermittelt werden kann. Dies ist bevorzugt dann der Fall, wenn die Scanpunkte auf der Oberfläche des Auges zumindest einen Teil der Pupille abdecken. Es ist weiter bevorzugt, wenn die Scanpunkte auf der Oberfläche des Auges das ganze Auge überdecken. Hierbei wird unter den Wörtern "Abdecken" oder "Überdecken" nicht verstanden, dass jeder einzelne Punkt abgescannt werden muss. Es reicht, wenn die Einhüllende der abgescannten Punkte größer ist als der Teil des Auges oder das ganze Auge.

Bei dem Verfahren wird ein Self-mixing-Effekt des vom Auge in die Laserquelle zurückgeworfenen gescannten Laserstrahls genutzt, um für die mindestens zwei Scanpunkte die optische Weglänge von der Laserquelle bis zu den mindestens zwei Scanpunkten auf der Augenoberfläche und eine Reflektivität des Auges an den mindestens zwei Scanpunkten zu bestimmen.

Bei dem Self-mixing-Effekt, auch "Laser Self Mixing" genannt, wird die von einem Laser ausgesandte kohärente Strahlung von einer Oberfläche gestreut und ein Teil dieser Strahlung gelangt wieder zurück in die Laserkavität, den optischen Resonator. Falls die zweifache Entfernung zum Streuer einem ganzzahligen Vielfachen der Wellenlänge entspricht, so befindet sich die zurückgestreute Strahlung in Phase mit der Strahlung in der Laserkavität. Damit addiert sie sich konstruktiv zu der dort befindlichen Strahlung, reduziert die Laserschwelle und steigert damit die Ausgangsleistung des Lasers. Wird nun die Entfernung des Streuers und damit die zurückgelegte optische Weglänge geändert, kommt es immer wieder in Abhängigkeit der Entfernung zu positiver oder negativer Interferenz innerhalb der Laserkavität, sodass die Leistung des Lasers in einem sinusförmigen Muster zwischen einem Strahlungsmaximum und einem Strahlungsminimum moduliert wird. Ähnliche Intensitätsmodulationen werden erreicht, wenn die Wellenlänge des Lasers moduliert wird oder wenn sich die Frequenz des rückgestreuten Laserlichts verändert, z.B. durch den Dopplereffekt, welcher bei der Reflektion an bewegten Objekten auftritt.

Wird nun die optische Strahlungsleistung durch eine Photodiode (engl.: monitoring photo diode) gemessen, kann anhand der Amplitudenänderung der Strahlungsleistung auf die Intensitätsänderung der zurückgestreuten Laserleistung geschlossen werden. Über eine Analyse der Anzahl der Schwingungen, z.B. durch Zählen der Nulldurchgänge oder der Maximalwerte, kann auch auf die Anzahl der Schwingungen, also Durchlaufvorgänge konstruktiver und destruktiver Interferenz, und damit bei bekannter Laser-Wellenlänge und Modulation auf die Entfernung und eventuelle Entfernungsänderungen zwischen Laserkavität und Streuer sowie auf die zum Laserstrahl parallele Geschwindigkeitskomponente des Streuers geschlossen werden. Zusätzlich zu Entfernung und Geschwindigkeit des Streuers kann die absolute Intensität des Rückstreusignals zu einer Berechnung der Reflektivität des Streuers genutzt werden. Dementsprechend weist der Laser bevorzugt eine integrierte Photodiode auf, welche die optische Strahlungsleistung des Lasers misst.

Es wird ein Oberflächenprofil des Auges ermittelt, indem mithilfe des Self-mixing-Effekts, welcher während eines Scannens des Laserstrahls über das Auge eine Modulation der Laserleistung bewirkt, eine Änderung einer optischen Weglänge von der Laserquelle zu einem aktuellen Scanpunkt auf der Augenoberfläche ermittelt wird. Dies wird vorliegend Umsetzungsvariante 2 genannt.

Die Modulation der Laserleistung ist eine sinusförmige Funktion der Änderung der optischen Weglänge von der Laserquelle zum aktuellen Scanpunkt auf der Augenoberfläche. Anhand der gemessenen Modulation kann somit die Änderung der optischen Weglänge ermittelt werden.

Hieraus folgt, dass anhand der bekannten geometrischen Anordnung der Laserquelle, des Reflexionselements und des Umlenkelements sowie anhand der bekannten optischen Eigenschaften des Umlenkelements für jeden Scanpunkt die Entfernung von der Laserquelle zur Oberfläche des Auges ermittelt werden kann. Da die geometrische Anordnung der Laserquelle, des Reflexionselements und des Umlenkelements bekannt sind, kann somit auch das Oberflächenprofil des Auges ermittelt werden, wodurch wiederum die Blickrichtung ermittelt werden kann.

Eine Änderung der optischen Weglänge bewirkt ein Durchlaufen mehrerer Extremstellen in der Laserleistungsmodulation durch eine konstruktive und destruktive Interferenz des Self-mixing-Effekts. Die relative Formänderung kann durch Analyse der Anzahl der durchlaufenen Extrema bestimmt werden.

Da die Wellenlänge der verwendeten Laserstrahlung bevorzugt im nahen Infrarot und damit in der Größenordnung eines Mikrometers liegt, bewirkt eine Änderung des Oberflächenprofils des Auges um z.B. 1 mm ein Durchlaufen von mehr als 2000 Extremstellen. Hieran erkennt man, dass durch diese Technik das Oberflächenprofil des Auges sehr genau vermessen werden kann. Alternativ zum vorstehend genannten Merkmal kann ein Gradient einer Modulation der Laserleistung zur Bestimmung eines Gradienten des Oberflächenprofils des Auges verwendet werden. Alternativ formuliert kann eine Steigung der Abstandsänderung mit einem zeitlichen Abstand der Extrema oder Nulldurchgänge der optischen Laserleistung verknüpft werden. Daher kann auch die Frequenz der zeitlichen Extrema als Maß für die Form und damit für die Blickrichtung herangezogen werden.

Gemäß dem beanspruchten Gegenstand werden eine Ausführungsform, wonach unterschiedliche Reflexivitäten unterschiedlicher Teile des Auges ermittelt werden, und die Ermittlung einer Änderung der optischen Weglänge miteinander kombiniert.

Dies hat den Vorteil, dass das Oberflächenprofil des Auges wesentlich genauer bestimmbar ist.

Gemäß einer bevorzugten Ausführungsform wird zusätzlich aufgrund von unterschiedlichen Reflektivitäten unterschiedlicher Teile des Auges an den mindestens zwei Scanpunkten die Blickrichtung des Auges ermittelt. Diese Ausführungsform wird vorliegend Umsetzungsvariante 1 genannt.

Die unterschiedlichen Teile des Auges sind die Sklera, welche auch Lederhaut genannt wird, die Iris, welche auch Regenbogenhaut genannt wird, und die Pupille. Aufgrund der unterschiedlichen Reflektivitäten von Sklera, Iris und Pupille, welche von dem Scan über das Auge erhalten wurden, kann vorteilhafterweise die Blickrichtung des Auges ermittelt werden.

Gemäß einer bevorzugten Ausführungsform wird die Blickrichtung eines Auges zusätzlich durch den "Rote-Augen-Effekt" ermittelt, indem ein Scanpunkt auf dem Auge gesucht wird, bei dem der Laserstrahl an der Retina des Auges reflektiert wird. Diese Ausführungsform wird vorliegend Umsetzungsvariante 1b genannt.

Bei dem bekannten "Rote-Augen-Effekt" findet eine Reflexion an der Retina statt. Ist die Lichtquelle näherungsweise auf der optischen Achse des Beobachters angeordnet, so sind Ein- und Ausfallswinkel der Strahlung gleich und ein starker Reflex ist beobachtbar. Für alle anderen Winkel erscheint die Pupille dunkel, da Einfalls- und Ausfallswinkel der Strahlung nicht übereinstimmen und die Strahlungsleistung durch Mehrfachreflexionen im Auge geschluckt wird.

Dieses Merkmal ermöglicht vorteilhafterweise eine genaue Bestimmung der Pupillenposition, da sich die Reflektivität der Pupille bzw. der dahinter sichtbaren Retina stark von der Reflektivität anderer Teile des Auges unterscheidet.

Gemäß einer weiteren bevorzugten Ausführungsform ist das Umlenkelement so angeordnet und eingerichtet, dass der Laserstrahl für jede Scanstellung des Reflexionselements vom Umlenkelement in jeweils dieselbe Richtung umgelenkt wird. Diese Richtung ist bevorzugt parallel zu mindestens einer Blickrichtung des Auges und weiter bevorzugt zu einer Blickrichtung geradeaus nach vorne. Die Blickrichtung geradeaus nach vorne ist dadurch definiert, dass die Blickrichtung im Wesentlichen senkrecht auf der Ebene des Brillenglases steht.

Diese Ausführungsform des HOE ist für die Umsetzungsvariante 1 und die Umsetzungsprinzip 2 bevorzugt.

Hierbei werden die scanwinkelabhängig einfallenden Laserstrahlen in parallel austretende Laserstrahlen umgelenkt. Da die Orientierung des Umlenkelements und des Reflexionselements durch das Brillengestell fest vorgegeben sind, ist somit auch die Austrittsposition des Laserstrahls aus dem Umlenkelement in Richtung des Auges fest. Eine weitere Koordinatensystemtransformation in Abhängigkeit des Abstandes des Auges vom Umlenkelement entfällt somit vorteilhafterweise.

Gemäß noch einer weiteren bevorzugten Ausführungsform ist das Umlenkelement so angeordnet und eingerichtet, dass für jede Scanstellung des Reflexionselements eine Augenstellung existiert, deren Blickrichtung parallel zur Ausbreitungsrichtung des vom Umlenkelement in dieser Scanstellung umgelenkten Laserstrahls ist. Bevorzugt sind in diesem Fall die Laserstrahlen vom Umlenkelement kommend konvergent und schneiden sich in einem Punkt im Inneren des Augapfels, bevorzugt im Drehpunkt des Auges.

Hierdurch wird vorteilhafterweise erreicht, dass für jeden Blickwinkel des Auges an derjenigen Stelle, an der der Blick das Umlenkelement trifft, ein Laserstrahl existiert, welcher parallel zur Sehachse verläuft. Ferner wird eine Erhöhung der Auflösung durch eine Beschränkung des Winkelbereichs erzielt.

Gemäß einer anderen bevorzugten Ausführungsform ist das Umlenkelement so angeordnet und eingerichtet, dass die Laserstrahlen, welche für unterschiedliche Scanstellungen des Reflexionselements vom Umlenkelement umgelenkt werden, divergent sind. Bevorzugt sind hierbei die Strahlen divergent und schneiden sich in einem Punkt, welcher auf einem Punkt der Blickrichtung liegt, bevorzugt auf einer Blickrichtung geradeaus nach vorne.

Diese Ausführungsform ist vor allem für die Umsetzungsvarianten 1 und 2 bevorzugt. Falls die Fläche des Umlenkelements kleiner als der gesamte Augenbereich ist, ermöglicht diese Realisierung vorteilhafterweise eine Aufweitung des Messbereichs, z.B. um auch Hautbereiche um das Auge herum zur Kalibrierung eines Referenzkoordinatensystems heranzuziehen. Das Blickfeld (engl.: Field of View) ist als der Winkelbereich definiert, welcher vom Auge aus gesehen werden kann. Der Augenbereich ist als Fläche definiert, in dem sich das Auge befinden kann.

Gemäß einer bevorzugten Ausführungsform ist das Umlenkelement so angeordnet und eingerichtet, dass das Umlenkelement mindestens zwei unterschiedliche Bereiche hat, wobei jeder Bereich des Umlenkelements die auf sie treffenden Laserstrahlen auf jeweils einen Punkt auf dem Auge umlenkt.

Bevorzugt sind die unterschiedlichen Bereiche disjunkt.

Bevorzugt weist das Umlenkelement eine Vielzahl von Bereichen auf, wobei die von der Vielzahl von Bereichen auf das Auge umgelenkten Punkte gleichmäßig über das Auge verteilt sind. Gemäß einer weiteren bevorzugten Ausführungsform ist ein solcher Bereich eine horizontal verlaufende Zeile oder Scanzeile des bevorzugt auf dem Brillenglas angeordneten Umlenkelements. Noch weiter bevorzugt weist das Umlenkelement eine Vielzahl von horizontal verlaufenden zeilenförmigen Bereichen auf, wobei jeder zeilenförmige Bereich oder mehrere zeilenförmige Bereiche die auf sie fallenden Laserstrahlen jeweils auf einen Punkt auf dem Auge umlenkt. Hierbei gibt es eine Eins-zu-eins-Zuordnung zwischen den horizontalen Bereichen und den Scanpunkten auf dem Auge, wobei jeder Bereich nur einem Punkt zugeordnet und jeder Punkt nur einem Bereich zugeordnet ist.

Diese Ausführungsform ist vorteilhaft, falls die Messqualität bei einem schnell scannenden System nicht ausreicht oder anders ausgedrückt, falls pro Messpunkt eine zu geringe Lichtausbeute vorliegt.

Die mindestens zwei unterschiedlichen Bereiche werden analog zu dem Eyebox-Konzept eines HOE definiert, welche ähnlich einem Parabolspiegel die Strahlen auf einen Punkt des Auges, die sogenannte Eyebox, fokussieren. Hierdurch wird vorteilhafterweise erreicht, dass die Auswerteelektronik vor allem bei der Umsetzungsvariante 1 länger über das empfangene Signal mitteln kann, wodurch das Signal-Rausch-Verhältnis verbessert wird.

Bei einem hochaufgelösten Projektionssystem mit beispielsweise 720 Zeilen ergeben sich ausreichend Messpunkte für eine Messung des Blickwinkels bei verhältnismäßig langer Verweilzeit an einem Messpunkt.

Die Projektionsvorrichtung für eine Datenbrille weist eine Laserquelle zum Aussenden eines Lichtstrahls auf. Ferner weist die Projektionsvorrichtung ein an einem Brillenglas der Datenbrille angeordnetes oder anordenbares Umlenkelement zum Umlenken des Laserstrahls in Richtung eines Auges des Nutzers und/oder zum Fokussieren des Laserstrahls auf. Darüber hinaus weist die Projektionsvorrichtung ein Reflexionselement zum Reflektieren des Laserstrahls auf das Umlenkelement und ein elektronisches Steuergerät auf.

Die Projektionsvorrichtung ist ausgeführt und das elektronische Steuergerät ist eingerichtet, ein Verfahren zum Ermitteln einer Blickrichtung eines Auges durchzuführen. Somit kann die Projektionsvorrichtung dieselben Vorteile wie das oben beschriebene Verfahren erzielen.

Die Datenbrille weist ein Brillenglas und eine oben beschriebene Projektionsvorrichtung auf, wobei das Umlenkelement am Brillenglas angeordnet ist. Die Datenbrille erreicht genauso wie die Projektionsvorrichtung dieselben Vorteile wie das oben beschriebene Verfahren.

Die Erfindung umfasst weiterhin ein Computerprogramm, das zur Durchführung der beschriebenen Schritte des Verfahrens eingerichtet ist, um mit diesem Computerprogramm das oben beschriebene Verfahren unter Verwendung der Projektionsvorrichtung durchführen zu können. Weiterhin umfasst die Erfindung ein maschinenlesbares Speichermedium, auf welchem ein solches Computerprogramm gespeichert ist, sowie ein elektronisches Steuergerät, das zur Durchführung der Schritte des beschriebenen Verfahrens unter Verwendung einer Projektionsvorrichtung eingerichtet ist. Ein solches elektronisches Steuergerät kann beispielsweise als Mikrocontroller in eine Projektionsvorrichtung oder Datenbrille integriert sein.

### Kurze Beschreibung der Zeichnungen

Ausführungsbeispiele der Erfindung sind in den Zeichnungen dargestellt und werden in der nachfolgenden Beschreibung näher erläutert.
Figur 1 zeigt eine schematische Darstellung einer Anordnung einer Datenbrille gemäß einer Ausführungsform und eines Auges eines Nutzers.
Figur 2 zeigt eine schematische Verteilung von Scanpunkten auf einem abgescannten Auge gemäß einer Ausführungsform des Verfahrens.
Fig. 3 zeigt ein schematisches Ablaufdiagramm eines Verfahrens gemäß einem Ausführungsbeispiel der Erfindung.
Figuren 4, 5, 6, 7, 8, und 9 zeigen jeweils eine schematische Darstellung einer Anordnung einer Datenbrille gemäß einer Ausführungsform und eines Auges eines Nutzers, wobei sich die physikalischen Eigenschaften des als HOE ausgeführten Umlenkelements unterscheiden.
Figur 10 zeigt eine Datenbrille mit einem Brillenglas gemäß der Ausführungsform der Figuren 8 und 9.
Figur 11 zeigt schematische Messdaten der optischen Weglänge in Abhängigkeit eines horizontalen Scanwinkels gemäß einer Ausführungsform des Verfahrens.
Figur 12 und 13 zeigen schematische Messergebnisse einer Ausführungsform des Verfahrens.
Figur 14 zeigt ein Blockschaltbild einer Ausführungsform einer Projektionsvorrichtung.

### Ausführungsbeispiele der Erfindung

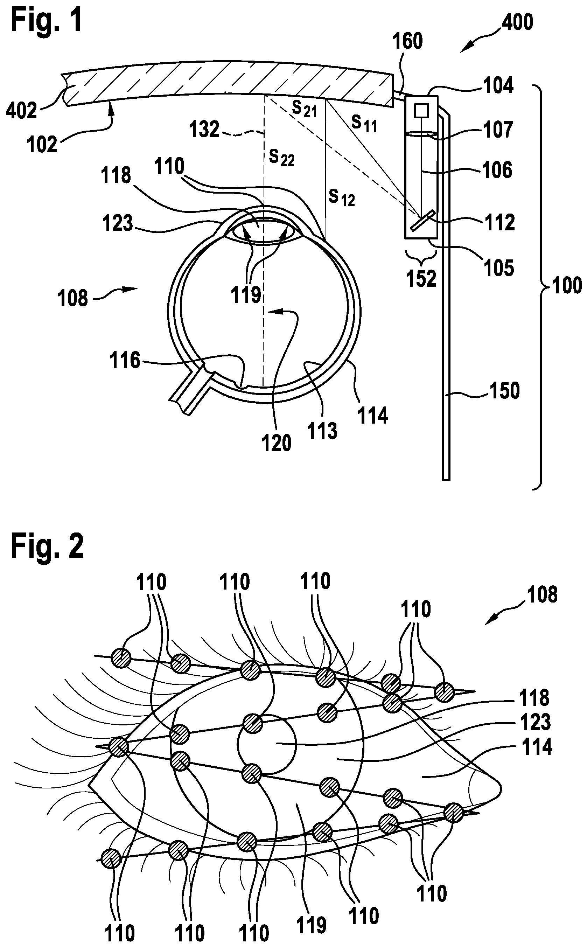

Figur 1 zeigt die prinzipielle Funktionsweise der Datenbrille 400, insbesondere anhand der Umsetzungsvariante 1. Die Datenbrille 400 weist ein Brillenglas 402 und eine Projektionsvorrichtung 100 auf. Die Projektionsvorrichtung 100 weist eine Scanneroptik 152 und ein Umlenkelement 102 auf, welches in dieser Ausführungsform als holografisches Element (HOE) ausgeführt ist. Das Umlenkelement 102 ist an dem Brillenglas 402 befestigt. Die Scanneroptik 152 ist in einem Gehäuse 105 angeordnet und weist eine Laserquelle 104, ein Kollimationselement 107, ein Reflexionselement 112 sowie ein nicht abgebildetes, nach dem Reflexionselement 112 angeordnetes Element zur Strahlformung und/oder Kollimation auf.

Ein von der Scanneroptik 152 emittierter Lichtstrahl 106 wird durch ein Austrittsfenster in Richtung des Umlenkelements 102 gesendet. Der vom Umlenkelement 102 umgelenkte Lichtstrahl 106 trifft sodann auf ein Auge 108 eines Nutzers. Die Scanneroptik 152 ist in einem am Brillengestell 160 und am Brillenbügel 150 befestigten Gehäuse 105 angeordnet.

Die Projektionsvorrichtung 100 kann ein Verfahren zum Ermitteln einer Blickrichtung eines Auges durchführen.

In einer weiteren Ausführungsform kann die Projektionsvorrichtung gleichzeitig ein Verfahren zur Erzeugung eines sichtbaren Bildes auf die Netzhaut und ein Verfahren zur Ermittlung einer Blickrichtung des Auges durchführen. Dabei können die optischen Funktionen des HOEs im Umlenkelement sich für verschiedene Wellenlängen unterscheiden.

Figur 1 zeigt zwei exemplarische Strahlengänge, einen ersten Strahlengang 131, bei dem der Laserstrahl 106 das Auge 108 im Bereich der Sklera 114 trifft und welcher eine erste Reflektivität aufweist sowie einen zweiten Strahlengang 132, bei dem der Laserstrahl 106 das Auge 108 im Bereich der Cornea 123 trifft und welche einen zweite Reflektivität aufweist. Der erste Strahlengang 131 entspricht einer ersten Spiegelstellung des Reflexionselementes 112, bei dem die Entfernung vom Reflexionselement 112 zum Umlenkelement 102 mit dem Bezugszeichen s₁₁ und die Entfernung vom Umlenkelement 102 zur Oberfläche des Auges 108, vorliegend zum Scanpunkt 110 auf der Sklera 114 mit dem Bezugszeichen s₁₂ bezeichnet wird. Der zweite Strahlengang 132 entspricht einer zweiten Spiegelstellung des Reflexionselementes 112, bei dem die Entfernung vom Reflexionselement 112 zum Umlenkelement 102 mit dem Bezugszeichen s₂₁ und die Entfernung vom Umlenkelement 102 zur Oberfläche des Auges 108, vorliegend zum Scanpunkt 110 auf der Cornea 123 mit dem Bezugszeichen s₂₂ bezeichnet wird. In der Abbildung der Figur 1 trifft in der zweiten Spiegelstellung des Reflexionselementes 112 der Laserstrahl 106 entlang der Blickrichtung 120 auf das Auge 108, sodass der Laserstrahl 106 zunächst auf die Cornea 123 und dann auf die Netzhaut 113 trifft.

Beim Scannen des Laserstrahls 106 wird dieser vergleichbar der Abtastung früherer Bildröhren in einem Zickzack-Muster über den Augapfel bewegt, d.h. mit schneller Zeilenfrequenz und geringerer Spaltenfrequenz, wie Figur 2 vereinfacht zeigt. In einem realen System kommen z.B. 1280 Scanpunkte 110 pro Zeile und 720 Zeilen zum Einsatz.

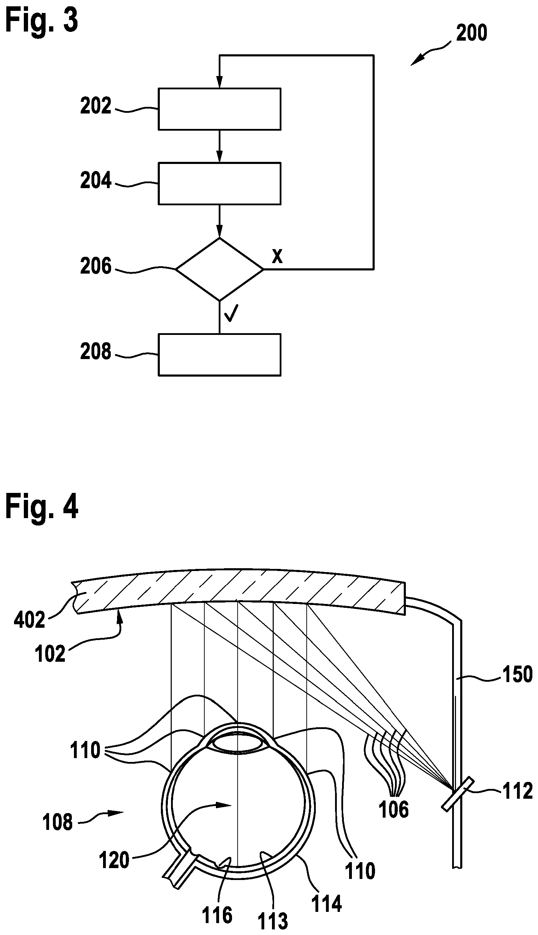

Figur 3 zeigt ein Verfahren 200 zum Ermitteln der Blickrichtung 120 des Auges 108. Das Verfahren 200 startet in Schritt 202 damit, dass das Reflexionselement 112 in eine erste Scanposition gebracht wird, bei der der Laserstrahl 106 auf einen ersten Scanpunkt 110 auf der Oberfläche des Auges 108 trifft. Im darauffolgenden Schritt 204 wird eine Reflektivität des Auges 108 am aktuellen Scanpunkt 110 die optische Weglänge von der Laserquelle 104 bis zum aktuellen Scanpunkt 110 auf der Augenoberfläche gemessen. Je nach Ausführungsform des Verfahrens 200 eignen sich hierzu unterschiedliche Arten von HOE als Umlenkelemente 102. Im nächsten Schritt 206 wird abgefragt, ob bereits alle Messpunkte 110 vermessen wurden oder nicht. Falls bereits alle Messpunkte 110 vermessen wurden, so fährt das Verfahren mit Schritt 208 fort, in dem die Blickrichtung 120 anhand der vermessenen Messpunkte 110 bestimmt wird. Falls noch nicht alle Messpunkte 110 vermessen wurden, so fährt das Verfahren mit Schritt 202 fort, in dem nun der nächste Scanpunkt am Reflexionselement 112 eingestellt wird. Ein Haken steht hierbei für eine Bejahung der Abfrage, ein Kreuz steht für eine Verneinung der Abfrage.

Die Bestimmung der Blickrichtung 120 unterscheidet sich auch bei den jeweiligen Verfahren der Umsetzungsvariante 1, 1b und 2.

Figur 4 zeigt eine Datenbrille 400 sowie das Auge 108, welches in derselben Position wie in Figur 1 ist. Das HOE des Umlenkelements 102 ist jedoch in der Ausführungsform der Figur 4 so ausgeführt, dass die Laserstrahlen 106 vom Umlenkelement 102 jeweils parallel zurückgeworfen werden. Hierbei sind die Laserstrahlen 106 jeweils parallel zum Brillenbügel 150. Die Richtung der Laserstrahl 106 entsprechend ebenfalls der Blickrichtung 120 des Auges 108, welches in der Figur 4 abgebildet ist.

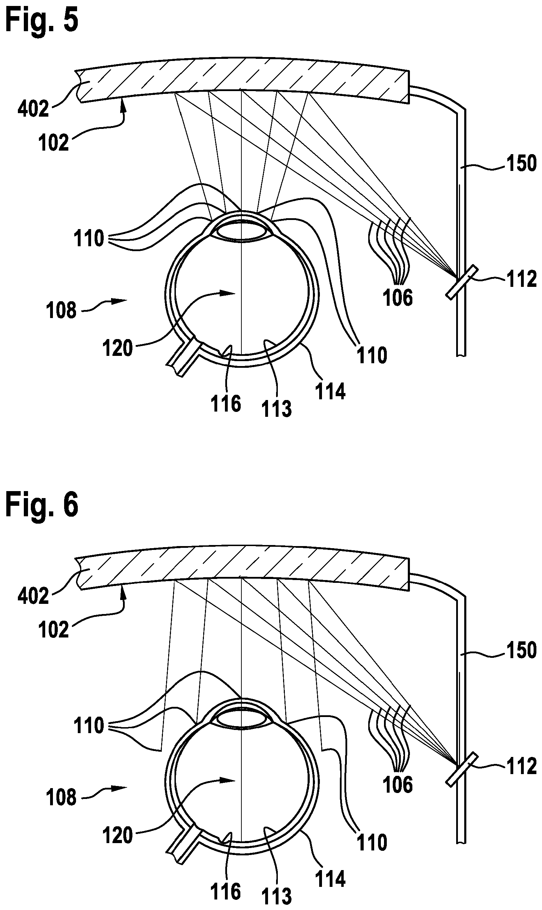

Dies ist besonders vorteilhaft für die Umsetzungsvarianten 1 und 2. Figur 5 zeigt ebenso wie Figur 4 eine Datenbrille 400 sowie das Auge 108. Im Unterschied zur Ausführungsform der Figur 4 ist das HOE des Umlenkelements 102 der Figur 5 jedoch so ausgeführt, dass die Laserstrahlen 106 vom Umlenkelement 102 konvergent zurückgeworfen werden, Dies ist besonders vorteilhaft für die Umsetzungsvariante 1b und eine Erhöhung der räumlichen Auflösung der Messung.

Figur 6 zeigt ebenso wie die Figuren 4 und 5 eine Datenbrille 400 sowie das Auge 108. Im Unterschied zu den Ausführungsformen der Figuren 4 und 5 ist das HOE des Umlenkelements 102 der Figur 6 jedoch so ausgeführt, dass die Laserstrahlen 106 vom Umlenkelement 102 divergent zurückgeworfen werden. Falls die Fläche des HOE kleiner als der gesamte Augenbereich ist, ermöglicht diese Realisierung die Aufweitung des Messbereichs, z.B. um auch Hautbereiche um das Auge herum zur Kalibrierung eines Referenzkoordinatensystems heranzuziehen.

Dies ist besonders vorteilhaft für die Umsetzungsvarianten 1 und 2.

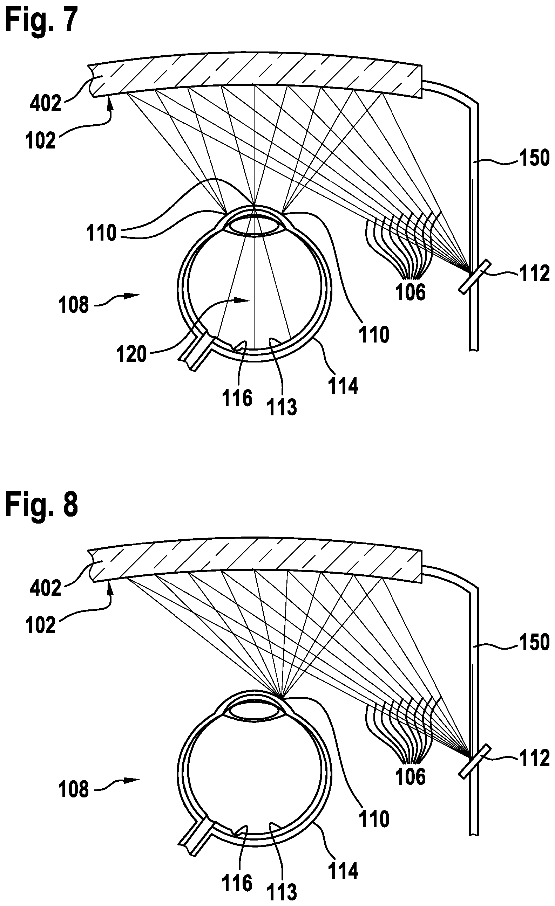

Figur 7 zeigt ebenso wie die Figuren 4, 5 und 6 eine Datenbrille 400 sowie das Auge 108. Im Unterschied zu den Ausführungsformen der Figuren 4, 5 und 6 ist das HOE des Umlenkelements 102 der Figur 7 jedoch so ausgeführt, dass mehrere Eyeboxen existieren, welche ähnlich einem Parabolspiegel die Strahlen an jeweils einem Scanpunkt 110 des Auges fokussieren.

Dies ist insbesondere dann vorteilhaft, wenn die Messqualität bei einem schnell scannenden System nicht ausreicht. Dadurch kann die Auswerteelektronik vor allem beim Umsetzungsprinzip 1 länger über das empfangene Signal mitteln und so das Signal-zu-Rausch-Verhältnis verbessern.

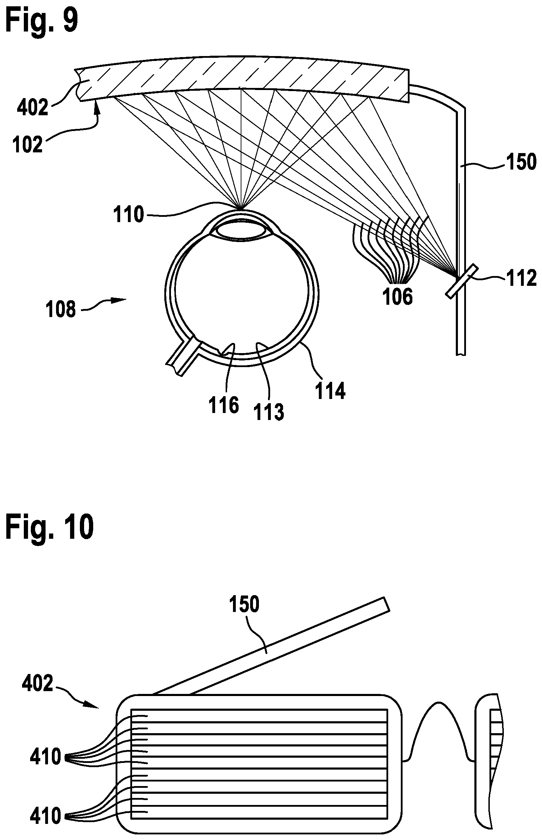

Eine weitere Variante dieses Ansatzes ist in Figuren 8, 9 und 10 gezeigt. Hierbei erstrecken sich die Eyebox-Regionen oder -Gebiete 410 auf dem Brillenglas 402 über jeweils eine gesamte Zeilenlänge einer Scan-Zeile, wie Figur 10 verdeutlicht. Überdies können sich die Eyebox-Regionen oder -Gebiete 410 über mehrere Scan-Zeilen erstrecken. Alle Laserstrahlen 106 innerhalb einer Eyebox-Region 410 werden jeweils auf eine feste Position auf dem Auge 108, die sogenannte Eyebox abgebildet, wie in Figur 8 z.B. für Eyebox-Region A und Figur 9 für z.B. Eyebox-Region G zu sehen ist.

Bei einem hochaufgelösten Projektionssystem mit beispielsweise 720 Zeilen ergeben sich somit ausreichend Scanpunkte 110 für eine Messung des Blickwinkels bei verhältnismäßig langer Verweilzeit an einem Messpunkt.

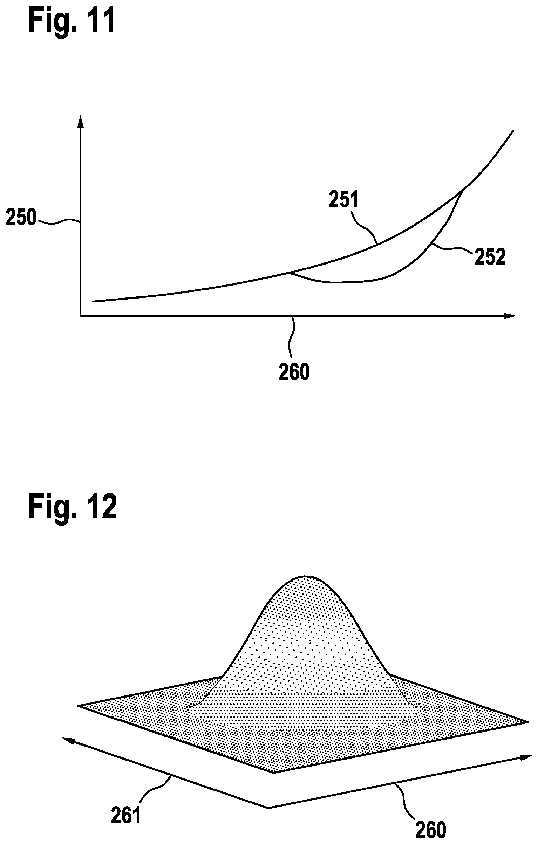

Figur 11 zeigt eine exemplarische Bestimmung einer optischen Weglänge 250 in Abhängigkeit vom horizontalen Scanwinkel 260 entsprechend Umsetzungsprinzip 2 unter Verwendung einer HOE-Funktion entsprechend Figur 4. Ein Beitrag 251 der Referenzebene der optischen Weglänge 250 beschreibt einen Einfluss der Entfernung zwischen Laser 104 und Umlenkelement 102. Darüber überlagert wird das Oberflächenprofil 252 des Auges 108, welches vorliegend ebenfalls als Entfernung gemessen wird. Aus dem Oberflächenprofil 252 des Auges 108 kann die Blickrichtung 120 des Auges 108 bestimmt werden.

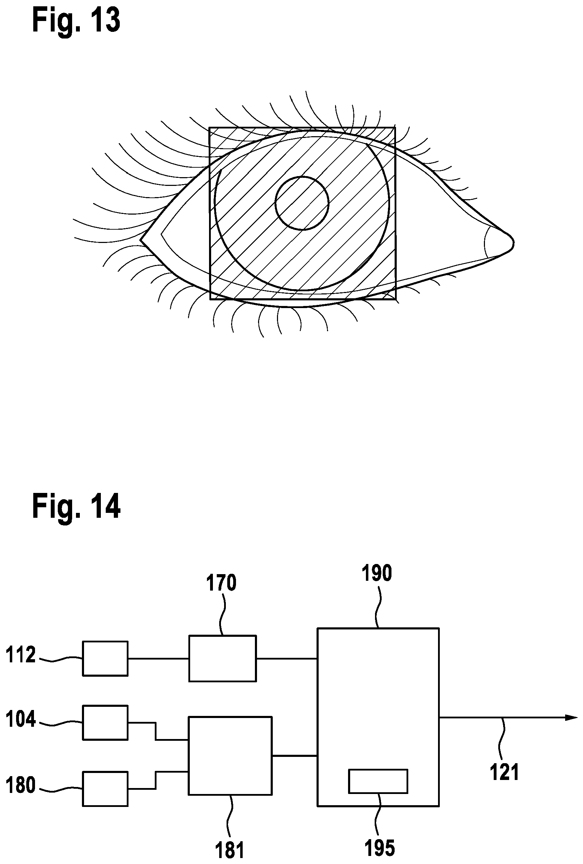

Hierbei können Auswerteverfahren direkt Parameter der Kurvenform wie Wendepunkte, Schnittpunkte und Extremstellen sein, es kann allerdings auch ein sogenanntes "template matching" zum Einsatz kommen, bei dem eine Modellfunktion an die gemessenen Daten angepasst wird. Eine skizzenhafte Darstellung der Ergebnisse einer Messung entsprechend Umsetzungsprinzip 1b zeigen die Figuren 12 und 13.

Figur 12 zeigt die reflektierte Laserintensität aufgetragen über den horizontalen Scanwinkel 260 und den vertikalen Scanwinkel 261. Figur 13 zeigt eine 2D-Projektion einer Intensität im Koordinatensystem des Auges 108. Durch Signalverarbeitungsverfahren kann anschließend die Mittenposition der Pupille 118 sowie eine Blickrichtung ermittelt werden.

Da Laser 104 und Photodiode in demselben Chip untergebracht sind, ist die Rote-Augen-Bedingung automatisch erfüllt, so dass Laserquelle und Detektor in derselben Achse liegen. Damit steigt die empfangene Lichtleistung an, sobald der Laserstrahl 106 durch die Pupille 118 zur reflektiven Retina 113 gelangt. Außerhalb der Pupille 118 wird nur wenig Lichtleistung zurückgestreut, da die Form des Auges 108, d.h. des Augapfels die meiste Leistung von der Detektionsachse, d.h. der Blickrichtung 120, weg reflektiert.

Falls die Intensitätsscans z.B. zu einem zweidimensionalen Array zusammengesetzt werden, so kann nach einer optionalen Filterung das Maximum bzw. der Mittelpunkt des Reflexionsmaximums bestimmt werden und daraus die Blickrichtung ermittelt werden.

Die Filterung kann ein räumlicher Glättungskernel sein, welcher eine Glättung der Signale bewirken, so dass eine Software, welche ein Maximum der Intensitätsverteilung sucht, nicht einen Ausreißer neben dem Hauptmaximum findet.

Figur 14 zeigt ein Blockschaltbild der Projektionsvorrichtung 100 für die Datenbrille 400. Die Projektionsvorrichtung 100 weist den Mikrospiegel 112 mit zugehöriger Ansteuerung 170, einen infraroten Laser 104 und eine Leistungsmonitoring-Photodiode 180 mit entsprechender Ansteuerung 181 auf. Die Photodiode 180 ist hierbei in den Laser 104 integriert. Zusätzlich gezeigt ist ein Mikroprozessor 190, also ein elektronisches Steuergerät, zur Ausführung des Programmcodes, also eines Computerprogramms, zur Berechnung eines Wertes 121 für die Blickrichtung 120 mit zugehörigem Speicher 195 für Kalibrierinformationen. Der berechnete Wert 121 für die Blickrichtung 120 kann anschließend an nachgelagerte Systemeinheiten weitergegeben werden.

## Patentansprüche

1. Verfahren (200) zum Ermitteln einer Blickrichtung (120) eines Auges (108),
wobei ein von einer Laserquelle (104) emittierter Laserstrahl (106) über ein Reflexionselement (112) und ein Umlenkelement (102) über mindestens zwei Scanpunkte (110) auf dem Auge (108) gescannt wird; ein Self-mixing-Effekt des vom Auge (108) in die Laserquelle (104) zurückgeworfenen gescannten Laserstrahls (106) genutzt wird, um für die mindestens zwei Scanpunkte (110) die optische Weglänge von der Laserquelle (104) bis zu den mindestens zwei Scanpunkten (110) auf der Augenoberfläche zu bestimmen,
**dadurch gekennzeichnet, dass**
ein Self-mixing-Effekt des vom Auge (108) in die Laserquelle (104) zurückgeworfenen gescannten Laserstrahls (106) genutzt wird, um zusätzlich die Reflektivität des Auges (108) an den mindestens zwei Scanpunkten (110) zu bestimmen, und ein Oberflächenprofil (140) des Auges (108) ermittelt wird, indem mithilfe des Self-mixing-Effekts, welcher während einem Scannen des Laserstrahls (106) über das Auge (108) eine Modulation der Laserleistung bewirkt, eine Änderung einer optischen Weglänge von der Laserquelle (104) zu einem aktuellen Scanpunkt (110) auf der Augenoberfläche ermittelt wird,
wobei die mindestens zwei Scanpunkte (110) auf der Sklera (114) und auf der Cornea (123) liegen.

2. Verfahren (200) nach Anspruch 1,
**dadurch gekennzeichnet, dass**
aufgrund von unterschiedlichen Reflektivitäten unterschiedlicher Teile des Auges (108) an den mindestens zwei Scanpunkten (110) die Blickrichtung des Auges (110) ermittelt wird.

3. Verfahren (200) nach Anspruch 1 oder 2,
**dadurch gekennzeichnet, dass**
die Blickrichtung des Auges (108) durch den "Rote-Augen-Effekt" ermittelt wird, indem ein Scanpunkt (110) gesucht wird, bei dem ein Einfallswinkel des Laserstrahls (106) mit der Blickrichtung des Auges (108) übereinstimmt.

4. Verfahren (200) nach einem der vorangegangenen Ansprüche,
**dadurch gekennzeichnet, dass**
das Umlenkelement (102) ein holografisch optisches Element (HOE) ist.

5. Verfahren (200) nach einem der vorangegangenen Ansprüche,
**dadurch gekennzeichnet, dass**
das Umlenkelement (102) so angeordnet und eingerichtet ist, dass der Laserstrahl (106) für jede Scanstellung des Reflexionselements (112) vom Umlenkelement (102) in jeweils dieselbe Richtung umgelenkt wird.

6. Verfahren (200) nach einem der vorangegangenen Ansprüche,
**dadurch gekennzeichnet, dass**
das Umlenkelement (102) so angeordnet und eingerichtet ist, dass für jede Scanstellung des Reflexionselements (112) eine Augenstellung existiert, deren Blickrichtung parallel zur Ausbreitungsrichtung des vom Umlenkelement (102) in dieser Scanstellung umgelenkten Laserstrahls (106) ist.

7. Verfahren (200) nach einem der vorangegangenen Ansprüche,
**dadurch gekennzeichnet, dass**
das Umlenkelement (102) so angeordnet und eingerichtet ist, dass die Laserstrahlen (106), welche für unterschiedliche Scanstellungen des Reflexionselements (112) vom Umlenkelement (102) umgelenkt werden, divergent sind.

8. Verfahren (200) nach einem der vorangegangenen Ansprüche,
**dadurch gekennzeichnet, dass**
das Umlenkelement (102) so angeordnet und eingerichtet ist, dass das Umlenkelement (102) mindestens zwei unterschiedliche Bereiche (410) hat, wobei jeder Bereich (410) des Umlenkelements (102) die auf sie treffenden Laserstrahlen (106) auf jeweils einen Punkt auf dem Auge (108) umlenkt.

9. Projektionsvorrichtung (100) für eine Datenbrille (400), wobei die Projektionsvorrichtung (100) folgende Merkmale aufweist:
eine Lichtquelle (104) zum Aussenden eines Laserstrahls (106);
ein an einem Brillenglas (402) der Datenbrille (400) angeordnetes oder anordenbares Umlenkelement (102) zum Umlenken des Laserstrahls (106) in Richtung eines Auges (108) des Nutzers und/oder zum Fokussieren des Laserstrahls (106); und
ein Reflexionselement (112) zum Reflektieren des Laserstrahls (106) auf das Umlenkelement (102), und ein elektronisches Steuergerät,
**dadurch gekennzeichnet, dass**
die Projektionsvorrichtung (100) ausgeführt und das elektronische Steuergerät eingerichtet ist, jeden Schritt des Verfahrens (200) nach einem der Ansprüche 1 bis 8 durchzuführen.

10. Datenbrille (400) mit folgenden Merkmalen:
einem Brillenglas (402); und
einer Projektionsvorrichtung (100) gemäß dem vorhergehenden Anspruch, wobei das Umlenkelement (102) am oder im Brillenglas (402) angeordnet ist.

11. Computerprogramm, welches eingerichtet ist, unter Verwendung einer Projektionsvorrichtung (100) nach Anspruch 9, jeden Schritt des Verfahrens (200) nach einem der Ansprüche 1 bis 8 durchzuführen.

12. Maschinenlesbares Speichermedium, auf welchem ein Computerprogramm nach dem vorangegangenen Anspruch gespeichert ist.

13. Elektronisches Steuergerät, welches eingerichtet ist, als Bestandteil der Projektionsvorrichtung (100) nach Anspruch 9, jeden Schritt des Verfahrens (200) nach einem der Ansprüche 1 bis 8 durchzuführen.

## Claims

1. Method (200) for ascertaining a direction of view (120) of an eye (108),
wherein a laser beam (106) emitted by a laser source (104) is scanned via a reflection element (112) and a deflection element (102) over at least two scan points (110) on the eye (108);
a self-mixing effect of the scanned laser beam (106), which is reflected off the eye (108) into the laser source (104), is used to determine, for the at least two scan points (110), the optical path length from the laser source (104) to the at least two scan points (110) on the eye surface,
**characterized in that**
a self-mixing effect of the scanned laser beam (106), which is reflected off the eye (108) into the laser source (104), is used to additionally determine the reflectivity of the eye (108) at the at least two scan points (110), and a surface profile (140) of the eye (108) is ascertained by virtue of the self-mixing effect, which causes a modulation of the laser power during a scanning of the laser beam (106) over the eye (108), being used to ascertain a change in an optical path length from the laser source (104) to a current scan point (110) on the eye surface,
wherein the at least two scan points (110) are located on the sclera (114) and on the cornea (123).

2. Method (200) according to Claim 1,
**characterized in that**
the direction of view of the eye (110) is ascertained on the basis of different reflectivities of different parts of the eye (108) at the at least two scan points (110).

3. Method (200) according to Claim 1 or 2,
**characterized in that**
the direction of view of the eye (108) is ascertained by the "red-eye effect" by virtue of searching for a scan point (110) at which an angle of incidence of the laser beam (106) coincides with the direction of view of the eye (108).

4. Method (200) according to any of the preceding claims,
**characterized in that**
the deflection element (102) is a holographic optical element (HOE).

5. Method (200) according to any of the preceding claims,
**characterized in that**
the deflection element (102) is arranged and configured such that the laser beam (106) is deflected in the same direction by the deflection element (102) for each scan position of the reflection element (112).

6. Method (200) according to any of the preceding claims,
**characterized in that**
the deflection element (102) is arranged and configured such that for each scan position of the reflection element (112) there exists an eye position, the direction of view of which is parallel to the direction of propagation of the laser beam (106) deflected in this scan position by the deflection element (102).

7. Method (200) according to any of the preceding claims,
**characterized in that**
the deflection element (102) is arranged and configured such that the laser beams (106) that are deflected by the deflection element (102) for different scan positions of the reflection element (112) are divergent.

8. Method (200) according to any of the preceding claims,
**characterized in that**
the deflection element (102) is arranged and configured such that the deflection element (102) has at least two different regions (410), wherein each region (410) of the deflection element (102) deflects the laser beams (106) incident thereon to respective points on the eye (108).

9. Projection device (100) for a pair of smartglasses (400), wherein the projection device (100) comprises the following features:
a light source (104) for transmitting a laser beam (106);
a deflection element (102) arranged or arrangeable on a lens (402) of the smartglasses (400) for deflecting the laser beam (106) in the direction of an eye (108) of the user and/or for focusing the laser beam (106); and
a reflection element (112) for reflecting the laser beam (106) onto the deflection element (102),
and an electronic controller, **characterized in that** the projection device (100) is embodied and the electronic controller is configured to perform each step of the method (200) according to any of Claims 1 to 8.

10. Smartglasses (400) with the following features:
a lens (402); and
a projection device (100) according to the preceding claim, wherein the deflection element (102) is arranged on or in the lens (402).

11. Computer program configured to perform each step of the method (200) according to any of Claims 1 to 8 using a projection device (100) according to Claim 9.

12. Machine-readable storage medium on which a computer program according to the preceding claim is stored.

13. Electronic controller configured as constituent part of the projection device (100) according to Claim 9 to perform each step of the method (200) according to any of Claims 1 to 8.

## Revendications

1. Procédé (200) pour déterminer une direction du regard (120) d'un œil (108),
dans lequel un faisceau laser (106) émis par une source laser (104) est amené à balayer au moins deux points de balayage (110) sur l'œil (108) par le biais d'un élément de réflexion (112) et d'un élément de déviation (102) ;
un effet d'auto-mélange du faisceau laser (106) balayé réfléchi par l'œil (108) dans la source laser (104) est utilisé pour déterminer, pour lesdits au moins deux points de balayage (110), la longueur du trajet optique de la source laser (104) auxdits au moins deux points de balayage (110) sur la surface de l'œil,
**caractérisé en ce que**
un effet d'auto-mélange du faisceau laser (106) balayé réfléchi par l'œil (108) dans la source laser (104) est utilisé pour déterminer en outre la réflectivité de l'œil (108) au niveau desdits au moins deux points de balayage (110), et **en ce qu'**un profil de surface (140) de l'œil (108) est déterminé en déterminant, à l'aide de l'effet d'auto-mélange, qui provoque une modulation de la puissance laser durant un balayage du faisceau laser (106) sur l'œil (108), une modification d'une longueur de trajet optique de la source laser (104) à un point de balayage actuel (110) sur la surface de l'œil,
dans lequel lesdits au moins deux points de balayage (110) se situent sur la sclérotique (114) et sur la cornée (123).

2. Procédé (200) selon la revendication 1,
**caractérisé en ce que**
la direction du regard de l'œil (110) est déterminée sur la base de différentes réflectivités de différentes parties de l'œil (108) au niveau desdits au moins deux points de balayage (110).

3. Procédé (200) selon la revendication 1 ou 2,
**caractérisé en ce que**
la direction du regard de l'œil (108) est déterminée par l'"effet yeux rouges" en recherchant un point de balayage (110) au niveau duquel un angle d'incidence du faisceau laser (106) coïncide avec la direction du regard de l'œil (108).

4. Procédé (200) selon l'une quelconque des revendications précédentes,
**caractérisé en ce que**
l'élément de déviation (102) est un élément optique holographique (HOE).

5. Procédé (200) selon l'une quelconque des revendications précédentes,
**caractérisé en ce que**
l'élément de déviation (102) est agencé et conçu de telle sorte que le faisceau laser (106) est dévié par l'élément de déviation (102) dans la même direction respective pour chaque position de balayage de l'élément de réflexion (112).

6. Procédé (200) selon l'une quelconque des revendications précédentes,
**caractérisé en ce que**
l'élément de déviation (102) est agencé et conçu de telle sorte que, pour chaque position de balayage de l'élément de réflexion (112), il existe une position de l'œil dont la direction du regard est parallèle à la direction de propagation du faisceau laser (106) dévié par l'élément de déviation (102) à cette position de balayage.

7. Procédé (200) selon l'une quelconque des revendications précédentes,
**caractérisé en ce que**
l'élément de déviation (102) est agencé et conçu de telle sorte que les faisceaux laser (106), qui sont déviés par l'élément de déviation (102) pour différentes positions de balayage de l'élément de réflexion (112), soient divergents.

8. Procédé (200) selon l'une quelconque des revendications précédentes,
**caractérisé en ce que**
l'élément de déviation (102) est agencé et conçu de telle sorte que l'élément de déviation (102) comporte au moins deux zones différentes (410), chaque zone (410) de l'élément de déviation (102) déviant les faisceaux laser (106) qui sont incidents sur celles-ci vers un point respectif sur l'œil (108).

9. Dispositif de projection (100) pour des lunettes de données (400), le dispositif de projection (100) présentant les caractéristiques suivantes :
une source de lumière (104) pour émettre un faisceau laser (106) ;
un élément de déviation (102), agencé ou pouvant être agencé sur un verre de lunettes (402) des lunettes de données (400), pour dévier le faisceau laser (106) en direction d'un œil (108) de l'utilisateur et/ou pour focaliser le faisceau laser (106) ; et
un élément de réflexion (112) pour réfléchir le faisceau laser (106) vers l'élément de déviation (102),
et une unité de commande électronique, **caractérisé en ce que** le dispositif de projection (100) est réalisé et l'unité de commande électronique est conçue pour mettre en œuvre chaque étape du procédé (200) selon l'une quelconque des revendications 1 à 8.

10. Lunettes de données (400) comprenant :
un verre de lunettes (402) ; et
un dispositif de projection (100) selon la revendication précédente, l'élément de déviation (102) étant agencé sur ou dans le verre de lunettes (402).

11. Programme d'ordinateur conçu pour mettre en œuvre, à l'aide d'un dispositif de projection (100) selon la revendication 9, chaque étape du procédé (200) selon l'une quelconque des revendications 1 à 8.

12. Support de stockage lisible par machine sur lequel est stocké un programme informatique selon la revendication précédente.

13. Appareil de commande électronique conçu pour mettre en œuvre, en tant que partie du dispositif de projection (100) selon la revendication 9, chaque étape du procédé (200) selon l'une quelconque des revendications 1 à 8.
